# EUROPEAN PATENT SPECIFICATION

(11) **EP 4 151 628 B1**
(45) Date of publication and mention of the grant of the patent: **15.04.2026**
(21) Application number: 21810887.6
(22) Date of filing: 30.08.2021
(51) Int. Cl.: C07D 401/04

(54) **PREPARATION METHOD FOR SYNTHESIZING CHIRAL NICOTINE FROM CHIRAL TERT-BUTYL SULFINAMIDE**
HERSTELLUNGSVERFAHREN ZUR SYNTHESE VON CHIRALEM NIKOTIN AUS CHIRALEM TERT-BUTYLSULFINAMID
MÉTHODE DE PRÉPARATION DE NICOTINE CHIRALE À PARTIR DE TERT-BUTYL SULFINAMIDE CHIRAL

(30) Priority: 28.07.2021 CN 202110860273
(43) Date of publication of application: 22.03.2023
(73) Proprietor: Shenzhen Zinwi Bio-tech Co., Ltd, Shenzhen, Guangdong 518107 (CN)
(72) Inventor: ZOU, Jun, Shenzhen, Guangdong 518107 (CN); ZOU, Yang, Shenzhen, Guangdong 518107 (CN); LIU, Meisen, Shenzhen, Guangdong 518107 (CN); LUO, Weixian, Shenzhen, Guangdong 518107 (CN)
(74) Representative: Dr. Gassner & Partner mbB
(86) International application number: PCT/CN2021/115386
(87) International publication number: WO 2023/004918

(56) References cited:
- WO-A1-2012/021712
- WO-A1-2017/201241
- WO-A1-2020/039209
- WO-A1-2020/108415
- WO-A2-2014/036502
- WO-A2-2018/081417
- CN-A- 109 053 525
- CN-A- 112 624 950
- CN-A- 112 679 421
- DEL CASTILLO ESTEFAN�A ET AL: "Enantioselective Synthesis of Nicotine via an Iodine-Mediated Hofmann-L�ffler Reaction", vol. 21, no. 3, 23 January 2019 (2019-01-23), US, pages 705 - 708, XP093104794, ISSN: 1523-7060, Retrieved from the Internet <URL:http://pubs.acs.org/doi/pdf/10.1021/acs.orglett.8b03909> DOI: 10.1021/acs.orglett.8b03909
- GWENDOLYN A. MARRINER, SEAN M. KERWIN: "An Improved Synthesis of (±)- N ′-Nitrosonornicotine 5′-Acetate", THE JOURNAL OF ORGANIC CHEMISTRY, AMERICAN CHEMICAL SOCIETY, vol. 74, no. 7, 3 April 2009 (2009-04-03), pages 2891 - 2892, XP055329311, ISSN: 0022-3263, DOI: 10.1021/jo9000417

## Description

### TECHNICAL FIELD

The present application relates to a technical field of chemical synthesis, and in particular to a preparation method for synthesizing a chiral nicotine from a chiral tert-butylsulfenamide.

### BACKGROUND

With the rapid development of the e-cigarette industry, nicotine, as one of the important active components of e-cigarette, is in increasing demand, and, in particular, nicotine with single configuration and optical activity has attracted extensive attention. However, there are few studies on the preparation methods of the chiral nicotine, most of which is basically obtained by chiral resolution, but reagents used in the chiral resolution is expensive, not conducive to industrial production.

China patent publication No. CN104341390A discloses a preparation method of the chiral nicotine, which uses a cyclic imine as a starting raw material, but requires expensive chiral catalysts to induce the formation of a chiral center. China patent publication No. CN11233829A discloses a preparation method of nicotine with optical activity, which uses chiral ligands containing nitrogen or phosphorus to prepare organic metal catalysts, and uses imine derivatives as the starting raw material to prepare the chiral nicotine. Similarly, the organic metal catalysts prepared by chiral ligands containing nitrogen or phosphorus are used as the chiral catalysts to induce the formation of the chiral centers, and the preparation method of the organic metal catalysts is complex and the production cost is high. The applicant found that the use of the chiral catalysts leads to more reaction steps for the whole synthesis of the chiral nicotine, resulting in the lower yield of the chiral nicotine.

The chiral tert-butylsulfenamide is a kind of raw material widely available and inexpensive, but there is no report on the synthesis of the chiral nicotine by using the chiral tert-butylsulfenamide as the raw material at present.

### BRIEF SUMMARY

To reduce reaction steps for preparing a chiral nicotine, the present application provides a preparation method for synthesizing a chiral nicotine from a chiral tert-butylsulfenamide.

In a first aspect, the present application provides the preparation method for synthesizing a chiral nicotine from chiral tert-butylsulfenamide, which is achieved by adopting technical solutions as follows.

The preparation method for synthesizing a chiral nicotine from chiral tert-butylsulfenamide includes steps as follow:
S1: condensing 3-pyridinecarboxaldehyde with the chiral tert-butylsulfenamide at the presence of a titanate to obtain a chiral 2-methyl-N-(pyridine-3-yl methylene)propane-2-sulfenamide, wherein a temperature of Step S1 is 30-70 °C;
S2: reacting the chiral 2-methyl-N-(pyridine-3-yl methylene) propane-2-sulfenamide with (1,3-dioxane-2-yl ethyl) magnesium bromide to obtain a chiral N-(3-(1,3-dioxane-2-yl)-1-(pyridine-3-yl) propylidene)-2-methyl propane-2-sulfenamide;
S3: cyclizing the chiral N-(3-(1,3-dioxane-2-yl)-1-(pyridine-3-yl) propylidene)-2-methyl propane-2-sulfenamide under an acidic condition to obtain a chiral 3-(3,4-dihydro-2H-pyrrol-2-yl) pyridine; and
S4: reducing and amine methylating the chiral 3-(3,4-dihydro-2H-pyrrol-2-yl) pyridine to obtain the chiral nicotine.

By adopting the above technical solution, in the present application, the chiral tert-butylsulfenamide is used as a starting raw material, condensed with 3-pyridinecarboxaldehyde, reacted with (1,3-dioxane-2-yl ethyl) magnesium bromide, cyclized under the acidic conditions, and finally reduced and amine methylated to obtain the chiral nicotine. A reaction route for synthesizing the chiral nicotine in the present application is shorter, and the raw materials are easily available and inexpensive, so that the production cost of the chiral nicotine can be reduced. In addition, reaction operations and processing operations in individual steps in the present application are simple, and a yield and an ee value of the chiral nicotine produced by the reaction are high. The preparation method for synthesizing the chiral nicotine from the chiral tert-butylsulfenamide according to the present application is an optimized method for synthesizing the nicotine with single configuration.

Preferably, in Step S1, a mole ratio of 3-pyridinecarboxaldehyde, the chiral tert-butylsul-fenamide to the titanate is 1:1:(1-3); and more preferably, the mole ratio of 3-pyridinecarbox-aldehyde, the chiral tert-butylsulfenamide and the titanate is 1:1:2.

In the present application, the chiral tert-butylsulfenamide can be (S)-tert-butylsulfenamide or (R)-tert-butylsulfenamide, which is determined by the configuration of the final product, that is, the chiral nicotine. When the chiral tert-butylsulfenamide is (S)-tert-butylsulfenamide, the chiral nicotine is (S)-nicotine; and when the chiral tert-butylsulfenamide is (R)-tert-butylsulfenamide, the chiral nicotine is (R)-nicotine.

Preferably, in Step S1, the titanate is one or more selected from the group consisting of tetraethyl titanate, tetrapropyl titanate and tetrabutyl titanate; and more preferably, the titanate is tetraethyl titanate.

Preferably, a solvent used in Step S1 is anhydrous tetrahydrofuran or dimethyl tetrahydrofuran; and preferably, the solvent used in Step S1 is anhydrous tetrahydrofuran.

Preferably, a temperature in Step S1 is 70°C.

In the present application, a reaction time of step S1 is 1.5-2.5h; and preferably, the reaction time of step S1 is 2h.

In the present application, the condensing in Step S1 occurs in a nitrogen atmosphere. The nitrogen atmosphere can improve activity of 3-pyridinecarboxaldehyde, reduce the occurence of other side reactions, and remain the configuration of the chiral tert-butylsulfenamide, thereby increasing the ee value and the yield of 2-methyl-N-(pyridine-3-yl methylene) propane-2-sulfenamide.

In the present application, after the condensing in Step S1, a post treatment is performed to obtain the chiral 2-methyl-N-(pyridine-3-yl methylene) propane-2-sulfenamide. The post treatment mainly includes subjecting to vigorous stirring in brine, filtrating, washing, liquid separating, extracting, water removing and solvent removing.

Preferably, in Step S2, the mole ratio of the chiral 2-methyl-N-(pyridine-3-yl methylene) propane-2-sulfenamide to (1,3-dioxane-2-yl ethyl) magnesium bromide is 1:(1.1-1.3); and more preferably, the mole ratio of the chiral 2-methyl-N-(pyridine-3-yl methylene) propane-2-sulfenamide and (1,3-dioxane-2-yl ethyl) magnesium bromide is 1:1.225.

In the present application, the solvent used in Step S2 is tetrahydrofuran.

In the present application, in Step S2, materials are added by: adding the chiral 2-methyl-N-(pyridine-3-yl methylene) propane-2-sulfenamide prepared in Step S1 into tetrahydrofuran, and then adding (1,3-dioxane-2-yl ethyl) magnesium bromide solution dropwise.

In the present application, the reacting of Step S2 include a reaction in nitrogen atmosphere and a reaction under a sealed condition. The temperature of the reaction in nitrogen atmosphere is -30°C, and the reaction time is 30min. The temperature of the reaction under the sealed conditions is 0°C, and the reaction time is 3h.

In the present application, after the reaction under the sealed condition in Step S2, the reaction solution is heated to 25°C, and then quenched. A reagent used in the quenching is a mixed solution of saturated NH₄Cl aqueous solution and ethyl acetate, in which a volume ratio of saturated NH₄Cl aqueous solution to ethyl acetate is 5:3.

In the present application, after the quenching in Step S2, a post treatment step is further performed to obtain the chiral N-(3-(1,3-dioxane-2-yl)-1-(pyridine-3-yl) propylidene)-2-methyl propane-2-sulfenamide. The post-treatment step mainly includes liquid separating, extracting, washing, water removing and solvent removing.

Preferably, in Step S3, pH of the acidic condition is 2-4; and preferably, the pH of the acidic condition is 3, and the reagent used is a solution of hydrochloric acid in methanol with HCl content of 20wt%.

In the present application, the chiral N-(3-(1,3-dioxane-2-yl)-1-(pyridine-3-yl) propylidene)-2-methyl propane-2-sulfenamide prepared in Step S2 is dissolved in tetrahydrofuran before it is cyclized in the hydrochloric acid methanol solution.

In the present application, the reaction temperature of the cyclizing in Step S3 is 20-30°C, the reaction time is 1.5-2.5h; and preferably, the reaction temperature of the cyclizing in Step S3 is 25°C, and the reaction time is 2h.

In the present application, a mixture containing the chiral 3-(3,4-dihydro-2H-pyrrol-2-yl) pyridine is obtained by the cyclizing in Step S3.

Preferably, in Step S4, a reducing agent used for the reducing is sodium borohydride. The chiral 3-(3,4-dihydro-2H-pyrrol-2-yl) pyridine is reduced to chiral demethylnicotine by the sodium borohydride.

Preferably, in Step S4, a mole ratio of the sodium borohydride to the chiral 3-(3,4-dihydro-2H-pyrrol-2-yl) pyridine is (1.5-2.5):1; and more preferably, the mole ratio of the sodium borohydride to the chiral 3-(3,4-dihydro-2H-pyrrol-2-yl) pyridine is 2:1.

In the present application, in Step S4, the reaction temperature of the reducing is (-5)-5°C, and a reaction time is 2.5-3.5h; and preferably, the reaction temperature of the reducing is 0°C, and the reaction time is 3h.

In the present application, in Step S4, the pH of system is adjusted to be alkaline before the amine methylating.

In the present application, in Step S4, the amine methylating uses cesium carbonate and methyl iodide.

In the present application, a mole ratio of cesium carbonate and methyl iodide is 1:(1.3-1.8):(1.1-1.3); and more preferably, the mole ratio of cesium carbonate and methyl iodide is 1:1.5:1.2.

In the present application, in Step S4, a reaction temperature of the amine methylating is 20-30°C, preferably 25°C, and a reaction time is 3h.

In the present application, in Step S4, after the amine methylating, the system is adjusted to a neutral pH by adding acid, extracted to obtain an organic phase, which is dried by Na₂SO₄, and vacuum concentrated to obtain a crude chiral nicotine. Finally, the crude chiral nicotine is subjected to atmospheric distillation purification for one time to obtain the chiral nicotine.

In summary, the embodiments of the present application have the beneficial effects as follow.

The present application provides a new method for synthesizing the chiral nicotine, which uses easily available and inexpensive chiral tert-butylsulfenamide as the starting raw materials. The chiral tert-butylsulfenamide has provided a chiral center, therefore, there is no need for expensive or complex chiral catalyst, and the cost of the raw materials is reduced. Further, the chiral tert-butylsulfenamide is condensed with 3-pyridinecarboxaldehyde, then reacted with (1,3-dioxane-2-yl ethyl) magnesium bromide, cyclized under the acid condition, and finally reduced and amine methylated to obtain chiral nicotine. The whole synthesis involves in a short reaction route, simple operations in each reaction step, the high yield and the ee value of the resulting chiral nicotine, and high purity that can be achieved only by one-time purification, so that the production cost of chiral nicotine is reduced.

### DETAILED DESCRIPTION

The present application is further described in detail below in combination with examples.

Raw materials used in the present application can be obtained through commercial sale. The raw materials not mentioned in the present application are purchased from Sinopharm Chemical Reagent Co., Ltd., unless otherwise stated.

Examples 1-15 provide a preparation method for synthesizing chiral nicotine from a chiral tert-butylsulfenamide. Examples 4 and 6 are reference examples.

Example 1 is described below as an example.

Example 1 provides a preparation method for synthesizing chiral nicotine from chiral tert-butylsulfenamide, in which the chiral tert-butylsulfenamide is S-tert-butylsulfenamide, and the chiral nicotine is S-chiral nicotine, and a synthetic route is shown as reaction formula 1:

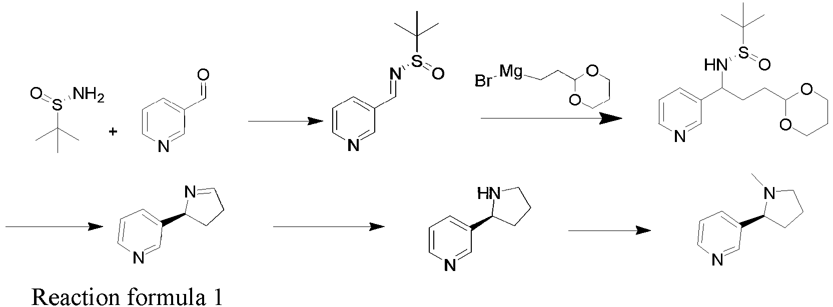

The specific preparation steps are shown as follows.

S1: in a nitrogen atmosphere, 106.7g (1mol, 1eq) 3-pyridinecarboxaldehyde, 121.7g (1mol, 1eq) (S)-tert-butylsulfenamide and 455.5g (2mol, 2eq) tetraethyl titanate were dissolved in 6L anhydrous tetrahydrofuran, and reacted at 70°Cfor 2h. After the reaction, a reaction solution was poured into 10L saturated salt water solution, stirred at 1000 rpm for 15 min, and filtered to obtain a filtrate and a filter cake. The filter cake was washed with 3L ethyl acetate, and the filtrate was collected, and separated to obtain a water layer. The water layer was extraced with 6L ethyl acetate-water (volume ratio of ethyl acetate to water is 2:1) for 3 times to obtain organic layers. The organic layers were combined, washed with 3L saturated salt water solution, dried by anhydrous Na₂SO₄ and vacuum concentrated to remove solvent to obtain a light yellow oily liquid of (S,E)-2-methyl-N-(pyridine-3-yl methylene) propane-2-sulfenamide.

S2: 8L tetrahydrofuran was added into (S,E)-2-methyl-N-(pyridine-3-yl methylene) propane-2-sulfenamide prepared by Step S1, and mixed uniformly. In the nitrogen atmosphere at -30°C, 2.45 L 0.5mol/L solution of (1,3-dioxane-2-yl ethyl) magnesium bromide in tetrahydrofuran was added dropwise (in which, (1,3-dioxane-2-yl ethyl) magnesium bromide is 1.225mol, 1.225eq), stirred and reacted at -30°C, 400rpm for 30min. Then, nitrogen was removed, and the reaction vessel was sealed, the reaction solution was stirred and performed at 0°C, 400rpm for 3h. After the reaction, the reaction solution was heated to 25°C, and a mixed solution of 0.5L saturated NH₄Cl water solution and 0.3L ethyl acetate were added for a quenching reaction. After the quenching reaction, the reaction solution was separated to obtain an organic layer and a water layer. The water layer was extracted with 10L ethyl acetate for 3 times, and separated. All the organic layers in the water layer were collected, combined, washed with 15L saturated salt water, dried with anhydrous magnesium sulfate, filtered and vacuum concentrated to obtain (S,E)-N-(3-(1,3-dioxane-2-yl)-1-(pyridine-3-yl) propylidene)-2-methyl propane-2-sulfenamide.

S3: 8L tetrahydrofuran was added into (S,E)-N-(3-(1,3-dioxane-2-yl)-1-(pyridine-3-yl) propylidene)-2-methyl propane-2-sulfenamide prepared by Step S2, and the system was adjusted to a pH of 3 by adding hydrochloric acid methanol solution with HCl content of 20wt% and reacted at 25°C for 2h to obtain a mixture containing (S)-3-(3,4-dihydro-2H-pyrrol-2-yl) pyridine.

S4: 75.66g (2mol, 2eq) sodium borohydride was added into the mixture containing (S)-3-(3,4-dihydro-2H-pyrrol-2-yl) pyridine prepared by Step S3, reacted at 0°C for 3h. (S)-3-(3,4-dihydro-2H-pyrrol-2-yl) pyridine is reduced to (S)-demethylnicotine, so as to obtain a mixing solution containing (S)-demethylnicotine. The pH of the mixing solution containing (S)-demethylnicotine to 9 with 4mol/L NaOH, and then 488.3g (1.5mol. 1.5eq) cesium carbonate and 170g (1.2mmol, 1.2eq) methyl iodide were added and reacted at 25°C for 3h, and the pH of the system was adjusted to 7 with 5mol/L HCl, and then the reaction solution was extracted with 15L saturated salt water and 15L dichloromethane to obtain an organic phase, which is collected and dried by adding anhydrous Na₂SO₄. The solvent was vacuum concentrated and evaporated to obtain a crude product of (S)-nicotine, which was atmospheric distillation purified to obtain (S)-nicotine, of which a yield is 72%, an ee value is 98%, and a purity is 98%.

Examples 2-3 differ from Example 1 only in that: in Step S1, an amount of the titanate is varied, as specifically shown in table 1.

**Table 1 Effect of the amount of the titanate on the yield of (S)-nicotine**

| No. | Equivalence quantity of titanate(eq) | Yield of (S)-nicotine (%) |
|---|---|---|
| Example 1 | 2 | 72 |
| Example 2 | 1 | 43 |
| Example 3 | 3 | 68 |

Example 4 differs from Example 1 only in that: in Step S1, the type of titanate is varied, as specifically shown in table 2.

**Table 2 Effect of the selection of titanate on the yield of (S)-nicotine**

| No. | Selected titanate | Yield of (S)-nicotine (%) |
|---|---|---|
| Example 1 | tetraethyl titanate | 72 |
| Example 4 | tetrabutyl titanate | 70 |

Examples 5-7 differ from Example 1 only in that: in Step S1, a reaction temperature is varied, as specifically shown in table 3.

**Table 3 Effect of the reaction temperature on the yield of (S)-nicotine**

| No. | Reaction temperature (°C) | Yield of (S)-nicotine (%) |
|---|---|---|
| Example 1 | 70 | 72 |
| Example 4 | 90 | 65 |
| Example 6 | 80 | 68 |
| Example 7 | 50 | 54 |

Example 8-9 differ from Example 1 only in that: in Step S1, the type of the solvent is varied, as specifically shown in table 4.

**Table 4 Effect of the solvent on the yield of (S)-nicotine**

| No. | Selection of the solvent | Yield of (S)-nicotine (%) |
|---|---|---|
| Example 1 | anhydrous tetrahydrofuran | 72 |
| Example 8 | dimethyl tetrahydrofuran | 70 |
| Example 9 | dichloromethane | 53 |

Examples 10-11 differ from Example 1 only in that: in Step S2, the amount of (1,3-dioxane-2-yl ethyl) magnesium bromide is varied, as specifically shown in table 5.

**Table 5 Effect of the amount of (1,3-dioxane-2-yl ethyl) magnesium bromide on the yield of (S)-nicotine**

| No. | Equivalence quantity of (1,3-dioxane-2-yl ethyl) magnesium bromide (eq) | the yield of (S)-nicotine (%) |
|---|---|---|
| Example 1 | 1.225 | 72 |
| Example 10 | 1.1 | 65 |
| Example 11 | 1.3 | 70 |

Example 12 differs from the Example 1 only in that: in Step S3, acid condition is varied, as specifically shown in table 6.

**Table 6 Effect of the acid conditions on the yield of (S)-nicotine**

| No. | the acid conditions | the yield of (S)-nicotine (%) |
|---|---|---|
| Example 1 | hydrochloric acid methanol solution with HCl content of 20wt% | 72 |
| Example 12 | 90wt% trifluoroacetic acid aqueous solution | 68 |

Examples 13-14 differ from the Example 1 only in that: in Step S4, reduction condition is varied, as specifically shown in table 7.

**Table 7 Effect of the reduction conditions on the yield of (S)-nicotine**

| No. | the reduction conditions | the yield of (S)-nicotine (%) |
|---|---|---|
| Example 1 | sodium borohydride | 72 |
| Example 13 | sodium triacetyl borohydride | 30 |
| Example 14 | sodium dithionite | 50 |

The Example 15 differs from the Example 1 only in that: in Step S1, (S)-tert-butylsulfenamide is replaced by (R)-tert-butylsulfenamide in equimolar. The yield of (R)-nicotine is 71%, the ee value is 98%, the purity is 98%.

### Comparative Example

The comparative Example 1 differs from the Example 1 only in that: in Step S1, the titanate is replaced by cesium carbonate in equimolar amount. The yield of (S)-nicotine is 28%, the ee value is 97%, the purity is 92%.

## Claims

1. A preparation method for synthesizing a chiral nicotine from a chiral tert-butylsulfenamide, **characterized by** comprising steps as follow:
Step S1: condensing 3-pyridinecarboxaldehyde with the chiral tert-butylsulfenamide at the presence of a titanate to obtain a chiral 2-methyl-N-(pyridine-3-yl methylene) propane-2-sulfenamide, wherein a temperature of Step S1 is 30-70°C;
Step S2: reacting the chiral 2-methyl-N-(pyridine-3-yl methylene) propane-2-sulfenamide with (1,3-dioxane-2-yl ethyl) magnesium bromide to obtain a chiral N-(3-(1,3-dioxane-2-yl)-1-(pyridine-3-yl) propylidene)-2-methyl propane-2-sulfenamide;
Step S3: cyclizing the chiral N-(3-(1,3-dioxane-2-yl)-1-(pyridine-3-yl) propylidene)-2-methyl propane-2-sulfenamide under an acidic condition to obtain a chiral 3-(3,4-dihydro-2H-pyrrol-2-yl) pyridine; and
Step S4: reducing and amine methylating the chiral 3-(3,4-dihydro-2H-pyrrol-2-yl) pyridine to obtain the chiral nicotine.

2. The preparation method for synthesizing the chiral nicotine from the chiral tert-butylsulfenamide according to claim 1, **characterized in that**, in Step S1, a mole ratio of 3-pyridinecarboxaldehyde, the chiral tert-butylsulfenamide and the titanate is 1:1:(1-3).

3. The preparation method for synthesizing the chiral nicotine from the chiral tert-butylsulfenamide according to claim 2, **characterized in that**, in Step S1, the mole ratio of 3-pyridinecarboxaldehyde, the chiral tert-butylsulfenamide and the titanate is 1:1:2.

4. The preparation method for synthesizing the chiral nicotine from the chiral tert-butylsulfenamide according to claim 1, **characterized in that**, in S1, the titanate is one or more selected from the group consisting of tetraethyl titanate, tetrapropyl titanate and tetrabutyl titanate.

5. The preparation method for synthesizing the chiral nicotine from the chiral tert-butylsulfenamide according to claim 1, **characterized in that**, a solvent used in Step S1 is one selected from a group consisting of anhydrous tetrahydrofuran and dimethyl tetrahydrofuran.

6. The preparation method for synthesizing the chiral nicotine from the chiral tert-butylsulfenamide according to claim 1, **characterized in that**, in Step S2, a mole ratio of the chiral 2-methyl-N-(pyridine-3-yl methylene) propane-2-sulfenamide and (1,3-dioxane-2-yl ethyl) magnesium bromide is 1:(1.1-1.3).

7. The preparation method for synthesizing the chiral nicotine from the chiral tert-butylsulfenamide according to claim 6, **characterized in that**, in Step S2, the mole ratio of the chiral 2-methyl-N-(pyridine-3-yl methylene) propane-2-sulfenamide and (1,3-dioxane-2-yl ethyl) magnesium bromide is 1:1.225.

8. The preparation method for synthesizing the chiral nicotine from the chiral tert-butylsulfenamide according to claim 1, **characterized in that**, in Step S4, a reducing agent used for the reducing is sodium borohydride.

9. The preparation method for synthesizing the chiral nicotine from the chiral tert-butylsulfenamide according to claim 8, **characterized in that** a mole ratio of sodium borohydride and the chiral 3-(3,4-dihydro-2H-pyrrol-2-yl) pyridine is (1.5-2.5):1.

## Patentansprüche

1. Herstellungsverfahren zur Synthese eines chiralen Nikotins aus einem chiralen tert-Butylsulfenamid, **dadurch gekennzeichnet, dass** es die folgenden Schritte umfasst:
Schritt S1: Kondensation von 3-Pyridincarboxaldehyd mit dem chiralen tert-Butylsulfenamid in Gegenwart eines Titanats, um ein chirales 2-Methyl-N-(pyridin-3-yl-methylen)propan-2-sulfenamid zu erhalten, wobei die Temperatur von Schritt S1 30-70°C beträgt;
Schritt S2: Umsetzen des chiralen 2-Methyl-N-(pyridin-3-yl-methylen)propan-2-sulfenamids mit (1,3-Dioxan-2-yl-ethyl)magnesiumbromid, um ein chirales N-(3-(1,3-Dioxan-2-yl)-1-(pyridin-3-yl)propyliden)-2-methyl-propan-2-sulfenamid zu erhalten;
Schritt S3: Cyclisierung des chiralen N-(3-(1,3-Dioxan-2-yl)-1-(pyridin-3-yl)propyliden)-2-methylpropan-2-sulfenamids unter einer sauren Bedingung, um ein chirales 3-(3,4-Dihydro-2H-pyrrol-2-yl)pyridin zu erhalten; und
Schritt S4: Reduktion und Aminmethylierung des chiralen 3-(3,4-Dihydro-2H-pyrrol-2-yl)-pyridins, um das chirale Nikotin zu erhalten.

2. Herstellungsverfahren zur Synthese des chiralen Nikotins aus dem chiralen tert-Butylsulfenamid nach Anspruch 1, **dadurch gekennzeichnet, dass** in Schritt S1 ein Molverhältnis von 3-Pyridincarboxaldehyd, dem chiralen tert-Butylsulfenamid und dem Titanat 1:1:(1-3) beträgt.

3. Herstellungsverfahren zur Synthese des chiralen Nikotins aus dem chiralen tert-Butylsulfenamid nach Anspruch 2, **dadurch gekennzeichnet, dass** in Schritt S1 das Molverhältnis von 3-Pyridincarboxaldehyd, dem chiralen tert-Butylsulfenamid und dem Titanat 1:1:2 beträgt.

4. Herstellungsverfahren zur Herstellung des chiralen Nikotins aus dem chiralen tert-Butylsulfenamid nach Anspruch 1, **dadurch gekennzeichnet, dass** in S1 das Titanat eines oder mehrere ist, ausgewählt aus der Gruppe bestehend aus Tetraethyltitanat, Tetrapropyltitanat und Tetrabutyltitanat.

5. Herstellungsverfahren zur Herstellung des chiralen Nikotins aus dem chiralen tert-Butylsulfenamid nach Anspruch 1, **dadurch gekennzeichnet, dass** in Schritt S1 ein Lösungsmittel verwendet wird, das aus der Gruppe ausgewählt ist, die aus wasserfreiem Tetrahydrofuran und Dimethyltetrahydrofuran besteht.

6. Herstellungsverfahren zur Herstellung des chiralen Nikotins aus dem chiralen tert-Butylsulfenamid nach Anspruch 1, **dadurch gekennzeichnet, dass** in Schritt S2 ein Molverhältnis des chiralen 2-Methyl-N-(pyridin-3-yl-methylen)-propan-2-sulfenamids und des (1,3-Dioxan-2-yl-ethyl)-magnesiumbromids 1:(1,1-1,3) beträgt.

7. Herstellungsverfahren zur Herstellung des chiralen Nikotins aus dem chiralen tert-Butylsulfenamid nach Anspruch 6, **dadurch gekennzeichnet, dass** in Schritt S2 das Molverhältnis des chiralen 2-Methyl-N-(pyridin-3-yl-methylen)-propan-2-sulfenamids und des (1,3-Dioxan-2-yl-ethyl)-magnesiumbromids 1:1,225 beträgt.

8. Herstellungsverfahren zur Synthese des chiralen Nikotins aus dem chiralen tert-Butylsulfenamid nach Anspruch 1, **dadurch gekennzeichnet, dass** in Schritt S4 ein zum Reduzieren verwendetes Reduktionsmittel Natriumborhydrid ist.

9. Herstellungsverfahren zur Synthese des chiralen Nikotins aus dem chiralen tert-Butylsulfenamid nach Anspruch 8, **dadurch gekennzeichnet, dass** das Molverhältnis von Natriumborhydrid und dem chiralen 3-(3,4-Dihydro-2H-pyrrol-2-yl)-pyridin (1,5-2,5):1 beträgt.

## Revendications

1. Procédé de préparation pour synthétiser une nicotine chirale à partir d'un tert-butylsulfénamide chiral, **caractérisé par** le fait de comprendre les étapes suivantes :
Étape S1 : condenser du 3-pyridinecarboxaldéhyde avec le tert-butylsulfénamide chiral en présence d'un titanate pour obtenir un propane-2-sulfénamide de 2-méthyl-N-(pyridine-3-ylméthylène) chiral, dans lequel une température de l'Étape S1 est de 30 à 70 °C ;
Étape S2 : faire réagir le propane-2-sulfénamide de 2-méthyl-N-(pyridine-3-ylméthylène) chiral avec du bromure de magnésium de (1,3-dioxane-2-yléthyle) pour obtenir un propane-2-sulfénamide de N-(3-(1,3-dioxane-2-yl)-1-(pyridine-3-yl)propylidène)-2-méthyle chiral ;
Étape S3 : cycliser le propane-2-sulfénamide de N-(3-(1,3-dioxane-2-yl)-1-(pyridine-3-yl)propylidène)-2-méthyle chiral à une condition acide pour obtenir une 3-(3,4-dihydro-2H-pyrrol-2-yl)pyridine chirale ; et
Étape S4 : réduire et réaliser une méthylation des amines de la 3-(3,4-dihydro-2H-pyrrol-2-yl)pyridine chirale pour obtenir la nicotine chirale.

2. Procédé de préparation pour synthétiser la nicotine chirale à partir du tert-butylsulfénamide chiral selon la revendication 1, **caractérisé en ce que**, à l'Étape S1, un rapport molaire de 3-pyridinecarboxaldéhyde, du tert-butylsulfénamide chiral et du titanate est de 1/1/(1 à 3).

3. Procédé de préparation pour synthétiser la nicotine chirale à partir du tert-butylsulfénamide chiral selon la revendication 2, **caractérisé en ce que**, à l'Étape S1, le rapport molaire de 3-pyridinecarboxaldéhyde, du tert-butylsulfénamide chiral et du titanate est de 1/1/2.

4. Procédé de préparation pour synthétiser la nicotine chirale à partir du tert-butylsulfénamide chiral selon la revendication 1, **caractérisé en ce que**, dans S1, le titanate est un ou plusieurs composés sélectionnés parmi le groupe constitué du titanate de tétraéthyle, du titanate de tétrapropyle et du titanate de tétrabutyle.

5. Procédé de préparation pour synthétiser la nicotine chirale à partir du tert-butylsulfénamide chiral selon la revendication 1, **caractérisé en ce qu'**un solvant utilisé à l'Étape S1 est un sélectionné parmi un groupe constitué du tétrahydrofurane anhydre et du tétrahydrofurane diméthylique.

6. Procédé de préparation pour synthétiser la nicotine chirale à partir du tert-butylsulfénamide chiral selon la revendication 1, **caractérisé en ce que**, à l'Étape S2, un rapport molaire du propane-2-sulfénamide de 2-méthyl-N-(pyridine-3-ylméthylène) chiral et du bromure de magnésium de (1,3-dioxane-2-yléthyle) est de 1/(1,1 à 1,3).

7. Procédé de préparation pour synthétiser la nicotine chirale à partir du tert-butylsulfénamide chiral selon la revendication 6, **caractérisé en ce que**, à l'Étape S2, un rapport molaire du propane-2-sulfénamide de 2-méthyl-N-(pyridine-3-ylméthylène) chiral et du bromure de magnésium de (1,3-dioxane-2-yléthyle) est de 1/1,225.

8. Procédé de préparation pour synthétiser la nicotine chirale à partir du tert-butylsulfénamide chiral selon la revendication 1, **caractérisé en ce que**, à l'Étape S4, un agent réducteur utilisé pour la réduction est le borohydrure de sodium.

9. Procédé de préparation pour synthétiser la nicotine chirale à partir du tert-butylsulfénamide chiral selon la revendication 8, **caractérisé en ce qu'**un rapport molaire de borohydrure de sodium et de la 3-(3,4-dihydro-2H-pyrrol-2-yl)pyridine chirale est de (1,5 à 2,5)/1.
